Europäisches Patentamt

European Patent Office

Office européen des brevets

(19)

(11) EP 1 004 892 A1

(12) EUROPEAN PATENT APPLICATION

(43) Date of publication:
31.05.2000 Bulletin 2000/22

(51) Int Cl.⁷: G01R 33/565

(21) Application number: 99309309.5

(22) Date of filing: 23.11.1999

(84) Designated Contracting States:
AT BE CH CY DE DK ES FI FR GB GR IE IT LI LU
MC NL PT SE
Designated Extension States:
AL LT LV MK RO SI

(30) Priority: 23.11.1998 US 198226

(71) Applicant: GENERAL ELECTRIC COMPANY
Schenectady, NY 12345 (US)

(72) Inventors:
• Ma, Jingfei
Waukesha, Wisconsin 53186 (US)

• Zhou, Xiaohong
Franklin, Wisconsin 53132 (US)
• McKinnon, Greame Colin
Hartland, Wisconsin 53029 (US)
• Sobering, Geoffrey S.
Madison, Wisconsin 53704 (US)

(74) Representative: Goode, Ian Roy et al
GE LONDON PATENT OPERATION,
Essex House,
12/13 Essex Street
London WC2R 3AA (GB)

(54) Compensating an MRI system for residual magnetization

(57) An MRI system includes a gradient compensation system which compensates imaging gradient waveforms for perturbations caused by residual magnetization. A calibration process (200 - 218) for measuring both the stagnant and transient components is performed to produce and store compensating data. The compensating data may be stored in a look-up table (220) or a polynomial may be fit to the compensating data for later use by the MRI system.

FIG. 12

## Description

The field of the invention is nuclear magnetic resonance imaging methods and systems. More particularly, the invention relates to the compensation for residual magnetization produced by magnetic field gradients in MRI systems.

When a substance such as human tissue is subjected to a uniform magnetic field (polarizing field $B_0$), the individual magnetic moments of the spins in the tissue attempt to align with this polarizing field, but precess about it in random order at their characteristic Larmor frequency. If the substance, or tissue, is subjected to a magnetic field (excitation field $B_1$) which is in the x-y plane and which is near the Larmor frequency, the net aligned magnetic moment, $M_z$, may be rotated, or "tipped", into the x-y plane to produce a net transverse magnetic moment $M_t$. A signal is emitted by the excited spins, and after the excitation signal $B_1$ is terminated, this signal may be received and processed to form an image.

The application of magnetic resonance to imaging, and to many of the techniques of localized spectroscopy, depends upon the use of linear magnetic field gradients to selectively excite particular regions and to encode spatial information within the NMR signal. During the NMR experiments, magnetic field gradient waveforms with particularly chosen temporal variations are used. Any departure from the application of ideal magnetic field gradient waveforms can, therefore, be expected to introduce image distortion, intensity loss, ghosting, and other artifacts. For example, imperfect rephasing of the nuclear spins and an attendant loss of signal occurs if the slice-selective magnetic field gradients are not balanced before and after the 180° pulse. This effect compounds in later spin echoes of multi-echo (Carr-Purcell-Mieboom-Gill) sequences. In addition, if the gradient field is not zero when it should be (due to residual magnetization after termination of a gradient pulse), the unintended phase dispersion can result in distorted spectra in chemical shift imaging (CSI) sequences as well as inaccurate spin-spin relaxation time ($T_2$) determination in multi-echo sequences. Those skilled in the art are thus concerned particularly about the accuracy with which time varying magnetic field gradients are produced.

One source of distortion in the production of magnetic field gradients can arise if the gradient fields couple to conductive structures within the polarizing magnet such as its cryostat (if the magnet is of the superconductive design), or the shim coil system, or the RF shield used to decouple the gradient coils from the RF coil. The induction of currents in these ambient structures are known as eddy currents. Due to eddy currents, one observes, typically an exponential rise and decay of the magnetic field gradient during and after, respectively, the application of a trapezoid current pulse to the gradient coil.

In U.S. Patent No. 4,698,591 entitled "A Method for Magnetic Field Gradient Eddy Current Compensation," a method is disclosed which uses an analog pre-emphasis filter in the gradient power supply to shape the current applied to the gradient coil in such a way that the eddy current induced gradient field distortions are reduced. The filter includes a number of exponential decay components and adjustable potentiometers which must be set during system calibration. A measurement technique is used prior to system calibration in which the impulse response of the uncorrected magnetic field gradient is measured and the potentiometer settings for the pre-emphasis filter are then calculated. Such techniques are described in U.S. patent Nos. 4,950,994; 4,698,591 and 4,591,789.

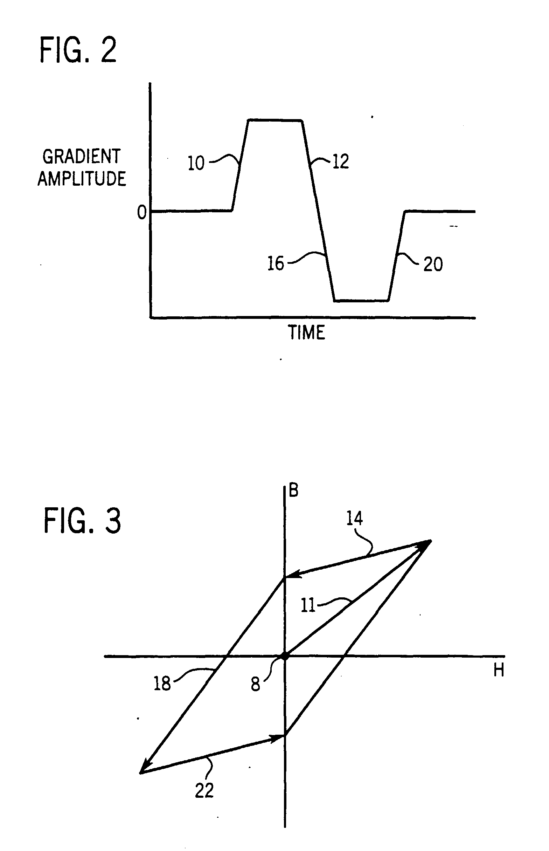

In iron-core permanent magnets or iron-core enhanced superconducting magnets, there exists another type of gradient-induced magnetic field perturbation. This perturbation, known as hysteresis, has not been well-studied, and generalized correction techniques have not been fully developed. To understand the hysteresis phenomenon, consider the effects of a bipolar gradient waveform shown in Fig. 2 and assume that the iron magnetization is in an initial state 8 shown in Fig. 3. The initial magnetization state is defined as the un-magnetized state, but in this case, it could be the state after the magnetic field is ramped up but before any gradients have ever been applied. During the first attack ramp, the current in the gradient coil, as well as the magnetic field H experienced by the iron core, is gradually increasing. As a result, the magnetic induction B increases with H, as indicated by curve 11 in Fig. 3. When the gradient is ramped down to zero at 12, however, the magnetic induction B does not return to zero. Instead, its dependence on the magnetic field is characterized by another curve 14. This phenomenon is know as hysteresis, and the remaining magnetic induction ($\Delta B$) is called remanence, or residual magnetization. If the gradient is further ramped down at 16 to a negative value, then the magnetic induction B follows curve 18. With subsequent gradient ramp 20, the H vs. B curve 22 ends with a negative residual magnetization ($-\Delta B$). Subsequent gradient pulses drive the magnetization in a loop, known as the hysteresis loop. It can be appreciated that the particular shape of the hysteresis loop will depend on the structure of the MRI system and that it typically will consist of curved lines.

The above analysis indicates that when a time-dependent magnetic field gradient pulse is used for imaging, a perturbation magnetic field $\Delta B$ can be generated in ferromagnetic materials. If the hysteresis effects are uncompensated, a number of image artifacts can be produced. For example, the residual magnetization induced by the phase-encoding gradient pulses in fast spin echo (FSE) can generate inconsistent phase errors in k-space data, leading to image blurring and ghosting.

This problem is addressed in U.S. Pat. No. 5,729,139. The proposed solution in this prior art patent is to correct the phase errors produced by residual magnetization. Ten specific methods for doing this are proposed and all require changes to the gradient pulse waveforms in the particular prescribed pulse sequence.

The present invention is a method and apparatus for reducing residual magnetization in an MRI system such that image artifacts are reduced. More particularly, the invention includes a method for calibrating an MRI system in which the residual magnetization produced by candidate imaging gradient waveforms to be used on the MRI system is measured and compensating changes to the candidate imaging gradient waveforms are determined. When candidate imaging gradients are subsequently used by the MRI system to acquire image data the compensating changes are made and the resultant gradient does not produce residual magnetization. Compensating changes include appending a compensation gradient lobe to the candidate imaging gradient waveform and changing the width of the candidate imaging gradient waveform.

The residual magnetization can be divided into two separate components, both of which can produce artifacts in MR images. The first is a stagnant hysteresis component and the second is a transient hysteresis component. The calibration process measures both hysteresis components and determines the compensating changes in candidate imaging gradient waveforms which will reduce the effects of both.

Yet another aspect of the present invention is an efficient method for compensating imaging gradient waveforms. The calibration process can produce a look-up table of compensating changes to be made to imaging gradient waveforms of various shapes. The appropriate compensating changes can be made to an imaging gradient just prior to its use in an image acquisition directly from this look-up table, or the compensating changes can be calculated by interpolating between values stored in the look-up table. In the alternative, a polynomial which defines the compensating changes as a function of imaging gradient waveform shape can be determined during the calibration process. When an image is to be acquired, the compensating changes for the imaging gradients are calculated using this polynomial.

Embodiments of the invention will now be described, by way of example, with reference to the accompanying drawings, in which:

Fig. 1 is a block diagram of an MRI system which employs the present invention;
Fig. 2 is a graphic representation of an exemplary imaging gradient waveform produced by the MRI system of Fig. 1;
Fig. 3 is a graphic illustration of the hysteresis curve produced by the imaging gradient waveform of Fig. 2;
Fig. 4 is a graphic representation of another exemplary imaging gradient waveform;
Fig. 5 is a graphic illustration of the hysteresis curve produced by the imaging gradient of Fig. 4;
Fig. 6 is a graphic representation of yet another exemplary imaging gradient waveform;
Fig. 7 is a graphic illustration of the hysteresis curve produced by the imaging gradient of Fig. 6;
Fig. 8 is a graphic illustration of a pulse sequence performed by the MRI system of Fig. 1 to determine a reset gradient that drives residual magnetization to zero;
Fig. 9 is a graphic illustration of hysteresis curve produced by the pulse sequence of Fig. 8;
Fig. 10 is a graphic illustration of a pulse sequence performed by the MRI system of Fig. 1 to produce a stagnant hysteresis compensation gradient lobe for an imaging gradient waveform;
Fig. 11 is a graphic illustration of a pulse sequence performed by the MRI system of Fig. 1 to adjust the width of an imaging gradient waveform to compensate for transient hysteresis;
Fig. 12 is a flow chart of a calibration process performed by the MRI system of Fig. 1 to compensate imaging gradient waveforms; and
Fig. 13 is an electrical block diagram of a gradient compensation system which forms part of the MRI system of Fig. 1.

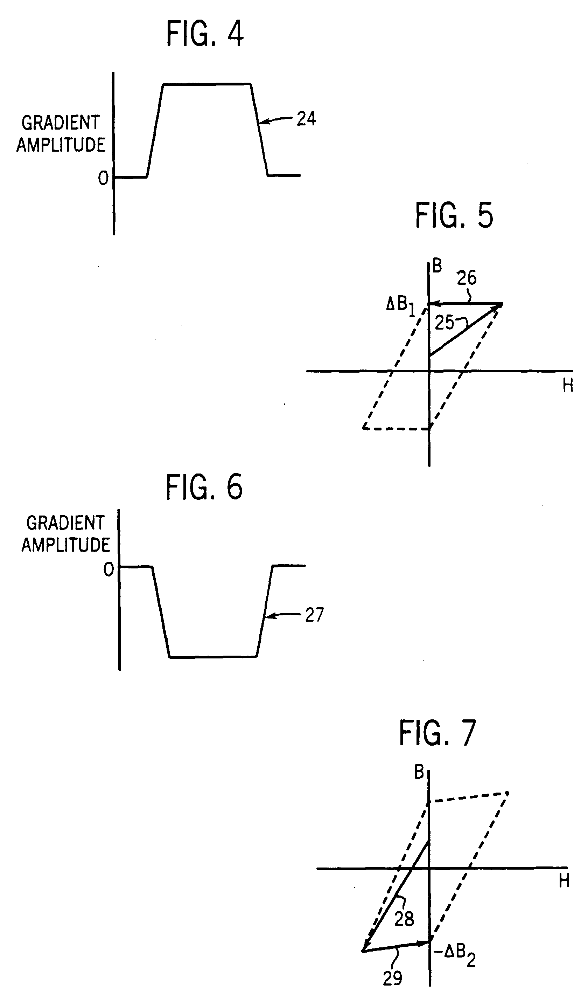

If a gradient waveform 24 is played out on an MRI system as shown in Fig. 4, the ferromagnetic structures in the MRI system will be driven along the hysteresis curve 25 and 26 in Fig. 5. A residual magnetization $\Delta B_1$ will remain at the completion of the gradient waveform 24. Similarly, if a negative gradient waveform 27 of the same amplitude is played out as shown in Fig. 6, the magnetization will be driven along the hysteresis curve 28 and 29 in Fig. 7, assuming the initial magnetization state is not at the origin. A residual magnetization $-\Delta B_2$ will remain at the completion of the waveform 27. The residual magnetization $\Delta B_1$ or $-\Delta B_2$ will produce a frequency offset from the Larmor frequency:

$$\Delta f_1 = \Delta B_1 \gamma$$

$$\Delta f_2 = \Delta B_2 \gamma \qquad (1)$$

where $\gamma$ is the gyromagnetic ratio of the spins. These frequency offsets can be measured by producing transverse magnetization from the spins with an RF excitation pulse applied after the gradient waveform 24 or 27 and by sampling the FID NMR signal that results. The Fourier transform of the acquired FID will produce a peak at the offset frequency $\Delta f_1$ or $\Delta f_2$.

If the hysteresis curve were perfectly symmetrical, $\Delta f_1$ would be equal to $-\Delta f_2$. This is not usually the case, however, and the offset frequency for zero magnetization is preferably calculated from the two meas-

ured offset frequencies:

$$\Delta f_0 = (\Delta f_1 - \Delta f_2)/2. \qquad (2)$$

This zero magnetization offset frequency is used in the following calibration procedure to produce a gradient "reset" pulse that drives residual magnetization to zero.

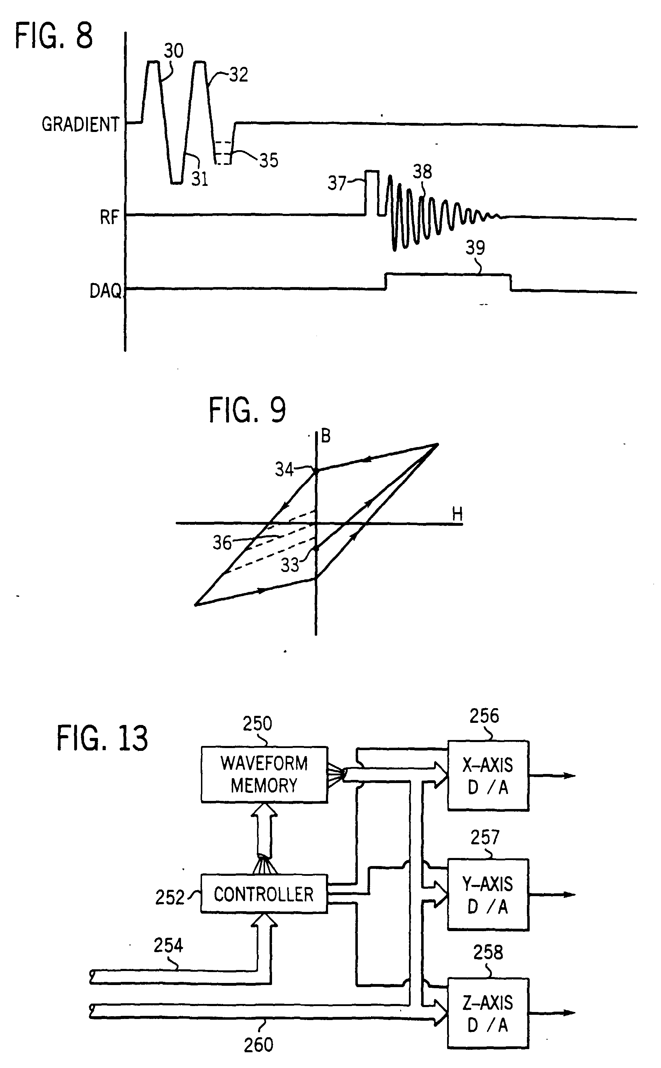

The reset gradient waveform is determined by performing the pulse sequence illustrated in Fig. 8 on the MRI system. The gradient waveform includes an odd number of lobes 30-32 with maximum amplitude, which alternate in polarity. As shown in Fig. 9, these gradients drive the magnetization of the ferromagnetic elements from an arbitrary residual magnetization indicated at point 33, around a hysteresis curve of maximum size possible, and ending at a positive maximum residual magnetization at point 34. A negative reset gradient lobe 35 is then applied with an amplitude that will drive the residual magnetization to zero as indicated by dotted line 36.

The size of the negative lobe 35 required to reset the residual magnetization to zero is determined in an iterative process using the pulse sequence of Fig. 8. The gradient waveform is played out with a selected reset lobe 35 and then a non-selective RF excitation pulse 37 is applied to produce transverse magnetization. The resulting FID signal 38 is acquired during a subsequent acquisition window 39 and its frequency is determined. The pulse sequence is repeated with a different amplitude reset lobe 35 until the measured frequency of the FID 38 is equal to the zero magnetization offset frequency $\Delta f_0$. This reset gradient is used to drive the residual magnetization to zero in the calibration measurements described below.

The perturbation in the magnetic field caused by residual magnetization can be divided into two components: a stagnant hysteresis component; and a transient hysteresis component. The stagnant component of residual magnetization remains for a long time without substantial temporal dependence after the gradient waveform is completed. The first correction method described below addresses this residual component by appending a compensation gradient lobe to the gradient waveform to drive the stagnant component to zero. The transient hysteresis component occurs concurrently with the application of the gradient waveform and alters its effective area. This transient hysteresis component is addressed by the second correction method discussed below by altering the area of the gradient waveform to offset this effect.

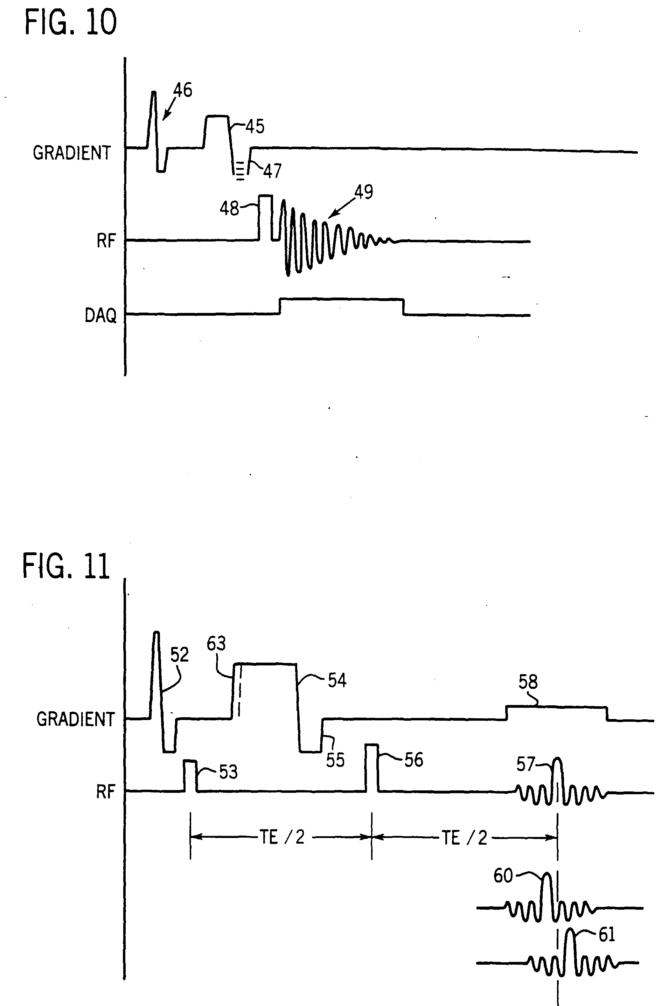

**Stagnant Hysteresis Component** - The measurement described above for determining the reset gradient also serves to determine the magnitude of a compensation gradient lobe for a gradient pulse of any given amplitude. Referring particularly to Fig. 10, the gradient waveform to be compensated is indicated by waveform 45, which might be, for example, a phase encoding gradient waveform. This "candidate" waveform is preceded by a reset gradient waveform 46 followed by a negative compensation gradient lobe 47 to drive the initial residual magnetization state to zero. The reset gradient waveform is a positive gradient lobe of maximum amplitude followed by a negative gradient lobe having the reset gradient amplitude discussed above or vice versa.

The amplitude of the compensation gradient lobe 47 is set to drive the positive residual magnetization produced by the candidate gradient waveform 45 to zero. This amplitude is determined in an iterative process similar to that described above, in which an RF excitation pulse 48 is applied and the frequency of the resulting FID signal 49 is determined. When the frequency of the FID 49 is the same as the previously measured zero magnetization offset frequency $\Delta f_0$, the candidate gradient waveform 45 is properly compensated for stagnant hysteresis.

It should be apparent that if the candidate gradient waveform is negative gradient the compensation gradient lobe will be positive in amplitude. If the candidate gradient waveform has both positive and negative gradient lobes (e.g. a slice select gradient) the proper compensation gradient lobe may be either positive or negative. This requires that the iterative process test values of the amplitude over a suitable range that will necessarily include the optimal amplitude.

While it is possible to measure the compensation gradient lobe for every imaging gradient waveform used in all imaging pulse sequences, it is also possible to pre-measure the compensation gradient required for a variety of gradient lobe amplitudes and widths and store them in a table. This process may be performed as part of the MRI system calibration. When a particular pulse sequence is subsequently prescribed, the proper compensation gradient amplitude is looked-up in the stored table and appended to the imaging gradient waveform. Another approach is to fit a polynomial to the measured compensation lobe amplitudes which express the relationship between candidate gradient lobe amplitude and width and the optimal compensation gradient lobe amplitude. At run time, therefore, the optimal compensation gradient lobe amplitudes are calculated using this polynomial function.

**Transient Hysteresis Component** - The transient hysteresis causes phase errors in spin magnetization that is in the transverse plane during and shortly after the candidate gradient waveform is played out. The transient hysteresis component is measured using the pulse sequence of Fig. 11 after the stagnant residual magnetization has been compensated as described above.

Referring particularly to Fig. 11, the residual magnetization is driven to zero by applying the reset gradient waveform 52 described above. Transverse magnetization is then produced by a non-selective 90° RF excitation pulse 53 and then the candidate gradient waveform 54 is applied. The candidate gradient waveform 54 has been compensated for stagnant hysteresis as described above by gradient lobe 55. A non-selective 180° RF echo pulse 56 is then applied at time TE/2 after the excitation pulse 53, and an NMR echo signal 57 is acquired in the presence of a readout gradient 58. The readout gradient is chosen such that its area equals the overall area of lobes 54 and 55. The readout gradient amplitude is kept to a minimum to minimize its own hysteresis effects.

Ideally, the echo signal 57 is precisely aligned with its peak occurring at the echo time TE after the rf excitation pulse 53. When the candidate gradient waveform 54 and 55 is applied, however, the transient hysteresis effect will cause the echo signal to shift in time as shown at 60 or 61. This phase offset is corrected by changing the width of the candidate pulse as indicated at 63. The pulse sequence of Fig. 11 is repeated in an iterative process with the width of candidate waveform 54 changed until the center of the echo signal 57 is precisely aligned at the ideal echo time TE.

This procedure can be used to adjust the width of every gradient waveform used on the MRI scanner, or as described above, a table or a polynomial can be produced during system calibration which is used to adjust each gradient waveform with a given amplitude and pulse width at run time. It should also be noted that this calibration procedure also compensates for any residual eddy currents having a short time constant.

## DESCRIPTION OF THE PREFERRED EMBODIMENT

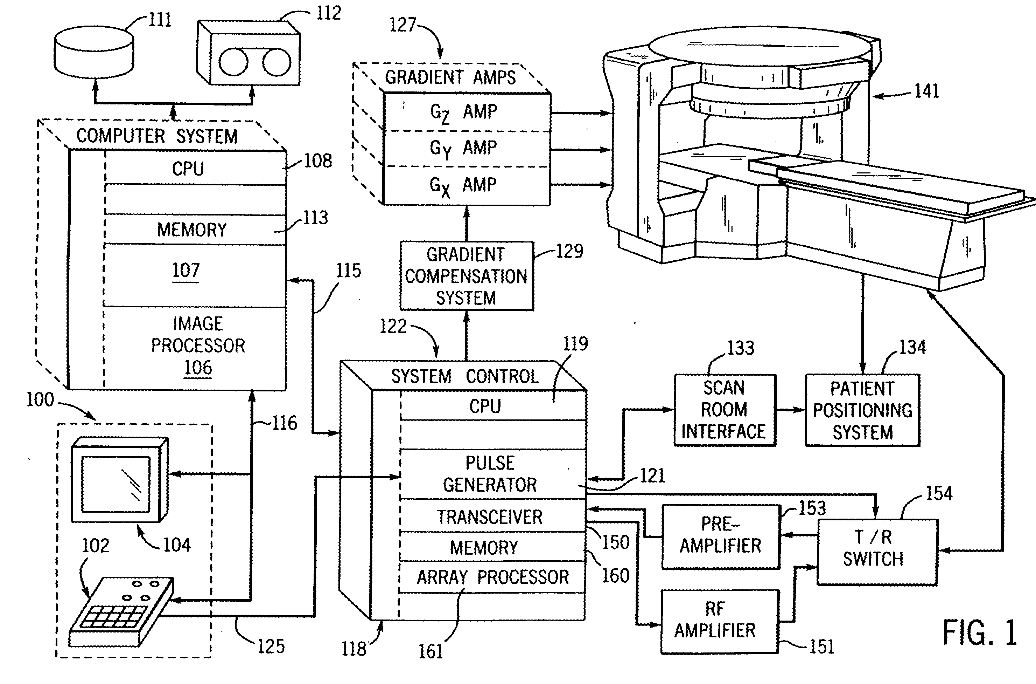

Referring first to Fig. 1, there is shown the major components of a preferred MRI system which incorporates the present invention. The operation of the system is controlled from an operator console 100 which includes a keyboard and control panel 102 and a display 104. The console 100 communicates through a link 116 with a separate computer system 107 that enables an operator to control the production and display of images on the screen 104. The computer system 107 includes a number of modules which communicate with each other through a backplane. These include an image processor module 106, a CPU module 108 and a memory module 113, known in the art as a frame buffer for storing image data arrays. The computer system 107 is linked to a disk storage 111 and a tape drive 112 for storage of image data and programs, and it communicates with a separate system control 122 through a high speed serial link 115.

The system control 122 includes a set of modules connected together by a backplane 118. These include a CPU module 119 and a pulse generator module 121 which connects to the operator console 100 through a serial link 125. It is through this link 125 that the system control 122 receives commands from the operator which indicate the scan sequence that is to be performed. The pulse generator module 121 operates the system components to carry out the desired scan sequence. It produces data which indicates the timing, strength and shape of the RF pulses which are to be produced, and the timing of and length of the data acquisition window. The pulse generator module 121 connects through a gradient compensation system 129 to a set of gradient amplifiers 127, to indicate the timing and shape of the gradient pulses to be produced during the scan. The pulse generator module 121 also connects to a scan room interface circuit 133 which receives signals from various sensors associated with the condition of the patient and the magnet system. It is also through the scan room interface circuit 133 that a patient positioning system 134 receives commands to move the patient to the desired position for the scan.

The gradient waveforms produced by the pulse generator module 121 are compensated by the system 129 as will be described in more detail below and applied to a gradient amplifier system 127 comprised of $G_x$, $G_y$ and $G_z$ amplifiers. Each gradient amplifier excites a corresponding gradient coil (not shown) which forms part of a magnet assembly 141. As is well known in the art, the gradient coils produce linear magnet field gradients used for position encoding acquired signals. The magnet assembly 141 also includes a polarizing magnet (not shown) and a whole-body RF coil (not shown). In the preferred embodiment the polarizing field is produced by a permanent magnet and associated iron core used to shape and direct the field as described in U.S. Pat. No. 5,652,517 entitled *"Magnet Assembly For MRI Apparatus"*. It is these elements which become magnetized by the gradient fields and which create the residual magnetization problem addressed by the present invention.

A transceiver module 150 in the system control 122 produces RF pulses which are amplified by an RF amplifier 151 and coupled to the RF coil in the magnet assembly 141 by a transmit/receive switch 154. The resulting signals radiated by the excited nuclei in the patient may be sensed by the same RF coil and coupled through the transmit/receive switch 154 to a preamplifier 153. The amplified NMR signals are demodulated, filtered, and digitized in the receiver section of the transceiver 150. The transmit/receive switch 154 is controlled by a signal from the pulse generator module 121 to electrically connect the RF amplifier 151 to the RF coil during the transmit mode and to connect the preamplifier 153 during the receive mode. The transmit/receive switch 154 also enables a separate RF calibration coil to be used in either the transmit or receive mode as will be described in more detail below.

The NMR signals picked up by the RF coil are digitized by the transceiver module 150 and transferred to a memory module 160 in the system control 122. When the scan is completed and an entire array of data has been acquired in the memory module 160, an array processor 161 operates to transform the data into an array of image data. This image data is conveyed through the serial link 115 to the computer system 107 where it is stored in the disk memory 111. In response to commands received from the operator console 100, this image data may be archived on external drive 112, or it may be further processed by the image processor 106 and conveyed to the operator console 100 and presented on the display 104.

For a more detailed description of the transceiver 150, reference is made to U.S. patent Nos. 4,952,877 and 4,992,736.

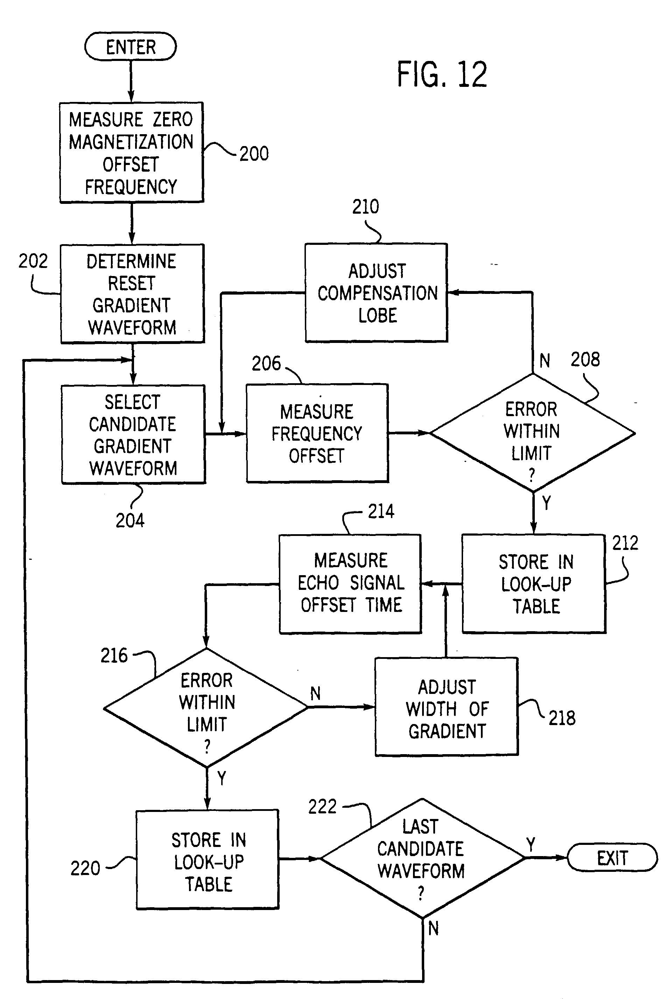

The MRI system is periodically calibrated using a procedure shown in Fig. 12. To make the measurements required by this procedure a calibration phantom is placed in the MRI system. The phantom supports a sample of MR active substance along one of the gradient axes and spaced from the system isocenter. For example, the sample is approximately 0.44 cc of 0.5M CuS04 doped water contained in a ¼ inch diameter acrylic tube. The sample serves as a source of NMR signals in the measurement pulse sequences used to calibrate the MRI system. Before performing the calibration procedure of the present invention the MRI system should be compensated to offset the effects of eddy currents using one of the well known methods.

Referring particularly to Fig. 12, the first step in the residual magnetization calibration procedure is to measure the zero magnetization offset frequency as indicated at process block 200. This step is described above with respect to equations 1-3 and it indicates the frequency of an NMR signal when no residual magnetization is present in the MRI system. The amplitude of the reset gradient waveform is then determined as indicated at process block 202. As discussed above, this step employs the pulse sequence of Fig. 8 to determine the shape of a reset gradient waveform that will drive the residual magnetization in the MRI system to zero.

A loop is then entered in which each candidate imaging gradient waveform is compensated for both stagnant hysteresis effects and transient hysteresis effects. Referring still to Fig. 12, a candidate gradient waveform is selected at process block 204 from a table which stores a list of gradient shapes, including amplitudes and pulse widths.

As indicated at process block 206, the pulse sequence of Fig. 10 is then performed to measure the frequency offset imposed by the candidate gradient waveform. If the measured frequency offset is within a preselected range of the zero magnetization offset as determined at decision block 208, the process proceeds to the next step. Otherwise, the compensation gradient lobe 47 (Fig. 10) is adjusted as indicated at process block 210 and the frequency offset measurement is repeated. This step is repeated until an optimal compensation gradient lobe is found for the candidate gradient waveform. This optimal compensation gradient lobe amplitude is stored in a look-up table as indicated at process block 212. During this calibration process the total net gradient area is maintained by adjusting the width of the candidate gradient waveform.

The next step indicated at process block 214 is to measure the echo signal time shift using the pulse sequence of Fig. 11. As explained above, the location of the peak in the echo signal 57 is detected and its offset from the echo time TE is determined by calculating a 1 D Fourier transformation and measuring the linear phase slope. If it is within a preset limited range from the echo time TE as determined at decision block 216, the compensation is complete. Otherwise, the width of the candidate gradient waveform is adjusted at process block 218 and the measurement of offset time is repeated at process block 214. The optimal adjustment that is finally obtained by this iterative process is stored in a look-up table as indicated at process block 220.

The above process is repeated for all gradient waveforms that are to be used on the MRI system. It is also repeated for each gradient axis x, y and z. When all candidate gradient waveforms have been compensated as indicated at decision block 222, the calibration process is completed.

It should be apparent that many variations are possible in the above-described calibration process. Rather than processing each and every candidate gradient waveform used in the MRI system, sample gradient waveforms at selected amplitudes and widths may be processed. The compensation values stored in the look-up table may subsequently be used directly to compensate the sample gradient waveforms when used in an imaging pulse sequence. In addition, other gradient waveforms may be compensated using values produced by interpolating between values stored in the look-up table.

Yet another alternative is to process sample gradient waveforms to produce a set of compensation values which indicate the optimal compensation as a function of candidate gradient waveform amplitude and width. These sample compensation values are then fit to a polynomial function which expresses the compensation values as a function of gradient amplitude and width.

When an image is to be acquired using the calibrated MRI system, the operator enters a prescribed scan into the MRI system. This prescription defines the scan parameters which include the particular gradient waveforms to be used during the imaging pulse sequence. Before being used, however, the gradient waveforms are compensated for residual magnetization using the compensation values calculated during the calibration process. This compensation can be performed in a number of ways.

In the preferred embodiment some of the imaging gradient waveforms are compensated before the scan starts. These include the slice selection gradient waveforms and the readout gradient waveforms that remain fixed during the scan. The compensation values for these gradient waveforms are read from the look-up table and used to modify their shape. This includes adding a compensation gradient lobe to the imaging gradient waveform and adjusting the width of the imaging gradient waveform. The compensated gradient waveforms are then stored in the pulse generator module 121 for use during the scan when they are played out to generate the imaging gradients.

Some of the imaging gradients are compensated as they are played out by the pulse generator module 121. The phase encoding gradient(s) in an imaging pulse sequence steps through many values (e.g. 128 or 256) during a scan and in the preferred embodiment these are compensated by the gradient compensation system 129 (Fig. 1) as they are played out.

Referring particularly to Fig. 13, the gradient compensation system 129 includes a waveform memory 250 which stores in digital form the look-up table calculated during the calibration process. This stored look-up table is used by a controller 252 to calculate a compensation waveform that is appended to the imaging gradient waveform when the controller 202 receives a command from the pulse generator module 121 over control bus 204. The digital values of this compensation waveform are applied to one or more A/D converters 256-258 through a data bus 260. The controller 252 enables the appropriate D/A converter(s) 256-258 and writes the digitized compensation waveform to them to produce analog versions of the compensation waveform at the output of one or more A/D converters 256-258. These outputs drive the respective x-axis, y-axis and z-axis gradient amplifiers 127.

When an imaging pulse sequence is performed by the MRI system of Fig. 1, the pulse generator module 121 produces the imaging gradient waveforms on data bus 260 and applies them to the appropriate D/A converter 256-258. The controller 252 is then signaled through control bus 254 to append a compensation waveform. The controller 252 produces the appropriate compensation waveform and applies it to the appropriate A/D converter(s) 256-258.

The complete gradient calibration process is comprised of the following steps:

1. Compensate for eddy currents;
2. Null stagnant residual magnetization while preserving the net gradient area of the candidate gradient waveforms; and
3. Adjust the width of the candidate gradient waveform to null the transient hysteresis effect and the transient eddy currents.

**Claims**

1. A method for calibrating an MRI system to reduce the effects of residual magnetization on acquired images, the steps comprising:

a) selecting a candidate gradient waveform (204) to be compensated;
b) measuring with the MRI system the residual magnetization effects (206,214) produced by the selected candidate gradient waveform;
c) determining compensation adjustment data (210,218) for the selected candidate gradient waveform which substantially reduces the residual magnetization effects;
d) repeating steps a), b) and c) for a plurality of different candidate gradient waveforms (222) used by the MRI system; and
e) storing the compensation adjustment data (212,220) for use by the MRI system when acquiring MR images.

2. The method as recited in claim 1 in which the compensation adjustment data are stored as compensation adjustment values in a look-up table.

3. The method as recited in claim 1 in which the compensation adjustment data are stored as a polynomial function which expresses compensation adjustments as a function of gradient waveform shape.

4. The method as recited in claim 1 in which step b) includes performing a pulse sequence with the MRI system using the selected candidate gradient waveform (45) to measure the shift in frequency of an NMR signal (49) caused by the candidate gradient waveform (45).

5. The method as recited in claim 4 in which the pulse sequence includes:

i) applying a reset gradient pulse waveform (46) to drive residual magnetization in the MRI system to substantially zero;
ii) applying the selected candidate gradient waveform (45);
iii) applying an rf excitation pulse (48) to produce an NMR signal (49); and
iv) acquiring the NMR signal.

6. The method as recited in claim 4 in which step c) includes:

i) appending a compensation gradient lobe (47) to the candidate gradient waveform (45); and
ii) adjusting the amplitude of the compensation gradient lobe (45) and repeating step b) until the measured shift in frequency is reduced below a preset limit;

wherein the resulting compensation gradient lobe amplitude is compensation adjustment data for the selected candidate gradient waveform.

**7.** The method as recited in claim 4 in which step b) also includes performing a second pulse sequence with the MRI system using the selected candidate gradient waveform (54) to measure the shift in echo time (TE) of an NMR echo signal(57,60,61) caused by the candidate gradient waveform (54).

**8.** The method as recited in claim 7 in which the second pulse sequence includes:

> i) applying a reset gradient pulse waveform (52) to drive residual magnetization in the MRI system to substantially zero;
> ii) applying an rf excitation pulse (53) to produce transverse magnetization;
> iii) applying the selected candidate gradient waveform (54);
> iv) applying an rf pulse (56) to invert the transverse magnetization and produce the NMR echo signal (57,60,61); and
> v) acquiring the NMR echo signal.

**9.** The method as recited in claim 7 in which step c) includes:

> adjusting the width of the selected candidate gradient waveform (54) and repeating step b) until the measured shift in echo time (TE) is reduced below a preset limit;
> wherein the resulting adjustment in width is compensation adjustment data for the selected candidate gradient waveform.

**10.** In an MRI system having a gradient system (127) for producing imaging magnetic field gradients during a scan in response to an imaging gradient waveform produced by a pulse generator (121), the improvement comprising:

> a gradient compensation system (129) coupled to the gradient system (127) and being operable to append a compensation gradient lobe (47) to the imaging gradient waveform (45) which drives residual magnetization in the MRI system to substantially zero after the image magnetic field gradient is produced.

**11.** The improvement as recited in claim 10 in which the gradient compensation system (129) includes means for producing a reset gradient (46) which is produced during the scan to drive residual magnetization in the MRI system to substantially zero.

**12.** A method for producing a reset gradient for an MRI system, the steps comprising:

> a) performing a pulse sequence with the MRI system which includes:

> i) producing a reset gradient waveform having a first lobe (32) and a second lobe (35);
> ii) producing an RF excitation pulse (37) to produce transverse magnetization; and
> iii) acquiring an NMR signal (38);

> b) determining the frequency of the NMR signal;
> c) changing the amplitude of the second reset gradient lobe (35); and
> d) repeating steps a) through c) until the frequency of the NMR signal is substantially equal to a zero magnetization frequency ($\Delta f_0$).

**13.** The method as recited in claim 12 in which the zero magnetization frequency ($\Delta f_0$) is determined by

> performing a pulse sequence with the MRI system to measure the frequency $\Delta f_1$ of an NMR signal acquired after a gradient pulse of one polarity is applied;
> performing another pulse sequence with the MRI system to measure the frequency $\Delta f_2$ of an NMR signal acquired after a gradient pulse of the opposite polarity is applied; and
> calculating the zero magnetization frequency as

$$\Delta f_0 - (\Delta f_1 - \Delta f_2)/2.$$

FIG. 1

EP 1 004 892 A1

9

## FIG. 2

## FIG. 3

FIG. 4

FIG. 5

FIG. 6

FIG. 7

11

FIG. 8

FIG. 9

FIG. 13

FIG. 10

GRADIENT

46

45

47

RF

48

49

DAQ

FIG. 11

GRADIENT

52

63

54

55

58

RF

53

56

57

TE / 2

TE / 2

60

61

# FIG. 12

ENTER

MEASURE ZERO
MAGNETIZATION
OFFSET
FREQUENCY — 200

DETERMINE
202 — RESET
GRADIENT
WAVEFORM

210
ADJUST
COMPENSATION
LOBE

206
SELECT
CANDIDATE
GRADIENT
WAVEFORM

MEASURE
FREQUENCY
OFFSET

N   208
ERROR
WITHIN
LIMIT
?

204

Y

214
MEASURE
ECHO SIGNAL
OFFSET TIME

212
STORE IN
LOOK-UP
TABLE

216
ERROR
WITHIN
LIMIT
?

N

ADJUST
WIDTH OF
GRADIENT — 218

Y

220 — STORE IN
LOOK-UP
TABLE

222
LAST
CANDIDATE
WAVEFORM
?

Y   EXIT

N

## EUROPEAN SEARCH REPORT

European Patent Office

Application Number

EP 99 30 9309

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 0 735 379 A (GE YOKOGAWA MEDICAL SYSTEMS, LTD.) 2 October 1996 (1996-10-02) * page 3, line 7 - page 5, line 53 * * page 6, line 29 - page 7, line 19 * * page 9, line 30 - page 11, line 44 * | 1-6,9-12 | G01R33/565 |
| D | & US 5 729 139 A | | |
| X | EP 0 818 689 A (GE YOKOGAWA MEDICAL SYSTEMS, LTD.) 14 January 1998 (1998-01-14) * column 1, line 50 - column 4, line 17 * * column 8, line 45 - column 11, line 22 * * column 14, line 8 - column 20, line 11 * | 1-6,9-12 | |
| A | EP 0 188 006 A (GENERAL ELECTRIC COMPANY) 23 July 1986 (1986-07-23) * page 6, line 16 - line 32 * * page 12, line 3 - page 14, line 8 * | 1,10,12 | |
| A | PATENT ABSTRACTS OF JAPAN vol. 16, no. 394 (C-0976), 21 August 1992 (1992-08-21) & JP 04 129531 A (TOSHIBA CORP), 10 April 1992 (1992-04-10) * abstract * | 1,10,12 | TECHNICAL FIELDS SEARCHED (Int.Cl.7) G01R |

The present search report has been drawn up for all claims

| Place of search | Date of completion of the search | Examiner |
|---|---|---|
| THE HAGUE | 21 February 2000 | Volmer, W |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 1 004 892 A1

## ANNEX TO THE EUROPEAN SEARCH REPORT
## ON EUROPEAN PATENT APPLICATION NO.

EP 99 30 9309

This annex lists the patent family members relating to the patent documents cited in the above-mentioned European search report.
The members are as contained in the European Patent Office EDP file on
The European Patent Office is in no way liable for these particulars which are merely given for the purpose of information.

21-02-2000

| Patent document cited in search report | | Publication date | Patent family member(s) | | Publication date |
|---|---|---|---|---|---|
| EP 735379 | A | 02-10-1996 | JP | 8322817 A | 10-12-1996 |
| | | | CN | 1136667 A | 27-11-1996 |
| | | | US | 5729139 A | 17-03-1998 |
| EP 818689 | A | 14-01-1998 | JP | 10075940 A | 24-03-1998 |
| | | | CN | 1171921 A | 04-02-1998 |
| EP 188006 | A | 23-07-1986 | US | 4665365 A | 12-05-1987 |
| | | | FI | 854524 A | 08-07-1986 |
| | | | JP | 1684395 C | 31-07-1992 |
| | | | JP | 3049257 B | 29-07-1991 |
| | | | JP | 61181950 A | 14-08-1986 |
| JP 04129531 | A | 30-04-1992 | NONE | | |

EPO FORM P0469

For more details about this annex : see Official Journal of the European Patent Office, No. 12/82

16